# EUROPEAN PATENT SPECIFICATION

(11) **EP 0 321 908 B1**
(45) Date of publication and mention of the grant of the patent: **30.03.1994**
(21) Application number: 88121238.5
(22) Date of filing: 19.12.1988
(51) Int. Cl.: A61L 2/20

(54) **Disinfectant vaporizing apparatus**
Gerät zur Verdampfung von Desinfektionsmitteln
Appareil pour la vaporisation de désinfectants

(30) Priority: 23.12.1987 JP 326287/87
(43) Date of publication of application: 28.06.1989
(73) Proprietor: SNOW BRAND MILK PRODUCTS CO., LTD., Sapporo-shi, Hokkaido 065 (JP)
(72) Inventor: Shibauchi Yoshito, Kawagoe-shi Saitama-ken (JP); Hatanaka, Koichi, Sayama-shi Saitama-ken (JP); Tanaka, Tatsuo, Irumagawa Sayama-shi Saitama-ken (JP)
(74) Representative: Hoffmeister, Helmut, Dr. Dipl.-Phys.

(56) References cited:
- EP-A- 0 114 619
- EP-A- 0 141 533
- GB-A- 392 044

## Description

### BACKGROUND OF THE INVENTION

The present invention relates to a gas generator for vaporizing a disinfectant by dropping it on a heating element, which apparatus is applied to such the technical field, of disinfection. For example, when a packing material including a container or the like is sterilized or disinfected by using disinfection gas, a material to be sterilized is sterilized by dropping a disinfection liquid on the heating element to vaporize, introducing the vaporized infection gas to the surface of the material to be sterilized by a heated carrier gas and condensing the carried disinfection gas on the surface of the material to be sterilized by a heated carrier gas and condensing the carried disinfection gas on the surface of the material to be sterilized.

Normally, hydrogen peroxide is used as a disinfection gas and a spray vaporization method. According to said spray vaporization method, it is necessary to provide a system for pressurizing hydrogen peroxide and a spray chamber. Some trouble in process such as blinding of nozzles and hunting of spray is probable to occur.

Japanese Patent Application 174235/86 (published under 63-11163) discloses a gas generator according to the preamble of claim 1. The known gas generator adopts a drop vaporization method in which hydrogen peroxide is vaporized by dropping it on the heating unit. A board heating type or a falling heating type is proposed as the construction of said heating unit.

With the board heating type unit, a stainless steel net is provided on a heat transfer block as an evaporation surface, a blow-off opening for a heated carrier air is provided above the evaporation surface parallel to the surface of the heat transfer block, the heated air is blown from the opening to promote the evaporation of hydrogen peroxide and the hydrogen peroxide gas is carried. A filter means is provided adjacent to the outlet of the vaporization chamber so as to prevent droplet splashes of hydrogen peroxide caused by increase of the carrier air or by the spheroidal phenomenon on the heated surface from being accompanied with the hydrogen peroxide gas.

Referring now to the falling heating type unit, a stainless steel net is provided as a vaporization surface in a vertical double heat pipe, a blow-off opening for heated carrier air is provided below said double heat pipes and the heated air is blown up along the interval between the double heat pipes. Other features are substantially same as those of the board heating type unit previously explained.

In general, the drop vaporization method presents some difficulties in controlling the rate of supply of the material to be sterilized to the sterilizing apparatus and the drying conditions of the material to be sterilized, unless the hydrogen peroxide gas not accompanied with the droplet splashes can be supplied on the surface of the material to be sterilized. That is, if the droplet splashes of hydrogen peroxide exists with the hydrogen peroxide gas, the gas density thereof becomes inhomogenuous, resulting in uneven sterilization of the surface to be sterilized.

In order to overcome the above disadvantages, there should be provided means for controlling the rate of supply of the material to be sterilized in accordance with the gas density or means for controlling the drying temperature, the drying velocity or the like in the drying process of the material to be sterilized. With this, however, the control of the sterilization becomes complex.

The above disadvantages have not yet overcome by the apparatus disclosed in said Japanese Patent Application 174235/86, because the board heating type unit thereof has the stainless net provided above the heat transfer block in the shape of the plate as the evaporation surface, the area to be heated extends horizontally; therefore occurs uneven heating and thermo difference in some heated surfaces. With this, the gas density also becomes uneven. Uniform supply of hydrogen peroxide for disinfection cannot be conducted.

Further, the hydrogen peroxide which is not evaporized due to inhomogenuous heating often accumulates at the bottom of the apparatus. Further there also occurs large heat loss caused by the large heating area. Additionally, the known filter means for preventing the drop splash of hydrogen peroxide accompanied with the hydrogen peroxide gas is not sufficient enough to make the vaporized hydrogen peroxide gas uniform.

Further according to the apparatus of said Japanese Patent Application 174235/86, the hot surface is extended vertically, and therefore it leaves same disadvantages as those of the board heating type unit.

### SUMMARY OF THE INVENTION

Accordingly the object of the present invention is to provide a disinfectant vaporizing apparatus or gas generator for dropping disinfection liquid on a heating unit and for providing uniform distribution of disinfection gas on a surface of a material to be sterilized.

According to the present invention, this is performed by the features of the characterizing clause of claims.

For the disinfectant, liquid disinfectant such as hydrogen peroxide, alcohol or the like can be used.

For the heated carrier gas, not only hot air but also inlet gas, mixed gas of inlet gas and air or the like can be used.

The apparatus according to the present invention has following advantages. Since the surface area of the evaporation part is smaller compared with the well-known unit, there are no uneven heating, no accumulation of the disinfectant such as hydrogen peroxide at the bottom of the evaporation part and no heating loss.

Further, since the surface area of the evaporation part is small, the control of the temperature of the evaporation part has become easier.

Additionally, since, when the surface area of the evaporation part is small and shield plates are provided above the evaporation part as a drop splash removing apparatus (9), the dropwise or splashwise disinfectant cannot rise and it combines each other into large drop, resulting in falling, uniform disinfection gas accompanied with the heated carrier gas can be supplied on the surface of the material to be sterilized.

Furthermore, the apparatus is compact enough as an evaporation means and it can easily be demounted in respect to maintenance and therefore has high high economical effect.

### BRIEF DESCRIPTION OF THE DRAWINGS

The above and the other objects of the invention will be seen by reference to the description taken in connection with the accompanying drawings, in which:
Fig. 1 is a perspective view including partial sectional view showing one embodiment according to the present invention using hydrogen peroxide as a disinfectant,
Fig. 2 is a sectional view showing the operation of the apparatus of Fig. 1,
Fig. 3 is a partial view of the evaporation unit, and
Fig. 4 is an explanational view with respect to the concaved cylindrical evaporation part.

### DESCRIPTION OF THE INVENTION

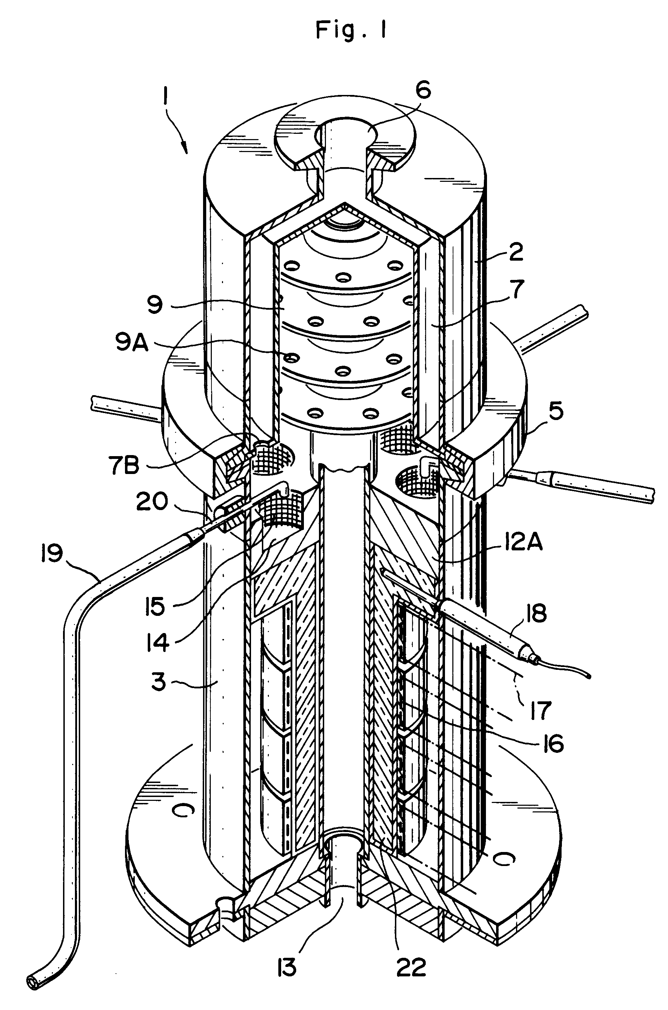

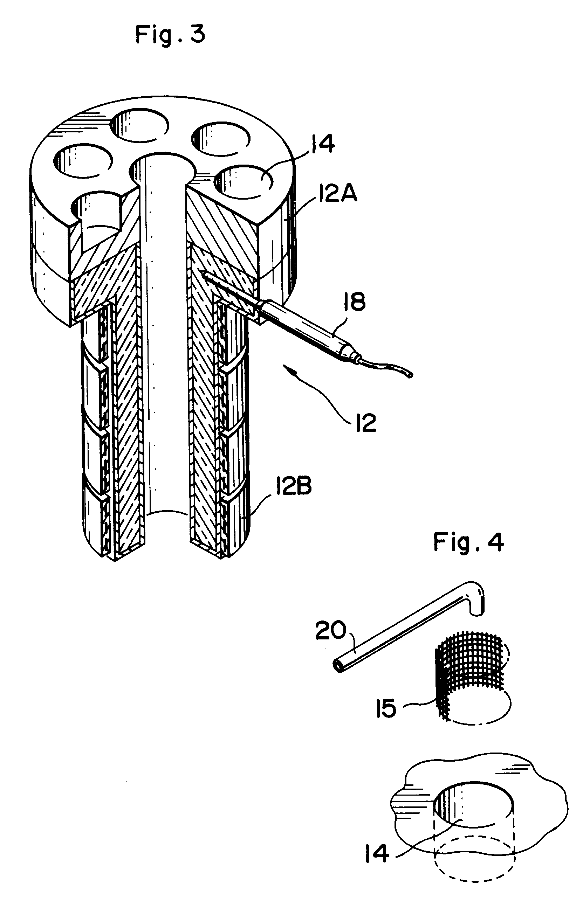

In Figure 1, 4 reference numeral 1 is a hydrogen peroxide vaporizing apparatus comprising an upper outer cylinder 2 and a lower outer cylinder 3. A flange 2A is provided in the lower surface of the upper outer cylinder 2 and a flange 3A is provided in the upper surface of the lower outer cylinder 3. A packing 4 is provided between the flanges. A ring 5 having concaved cross section is removably mounted to cover the flanges. An inlet port 6 is arranged on the upper portion of the upper outer cylinder 2. An inner cylinder 7 is provided inside the upper outer cylinder 2, which inner cylinder 7 has a flange 7A at the lower portion thereof and the periphery of the flange 7A is arranged to fit between a cutout portion 2B of the flange 2A and the inner periphery surface of the flange 3A. A hole 7B is provided in the flange 7A, with this, the carrier hot air is discharged to a vaporization chamber 8.

The vaporization chamber 8 of hydrogen peroxide is determined by the flange 7A, an evaporation unit 12 and the lower outer cylinder 3. The profile of the vaporization chamber of the present invention is not limitted to the above construction. Shield disks 9 are provided in an upper inner tube 10 inside the inner cylinder 7 and a stairs construction is formed by the plural shield disks 9. Plural holes 9A are provided on the shield disks 9, which holes adjacent to each other are arranged staggeringly and the plural holes of the plural shield disks are arranged not to overlap each other to the vertical direction.

The arrangement of the plural holes adjacent to each other and the arrangement of the holes of the plural shield disks are not limitted to the above described arrangement and any construction according to claim 1 in which the mix gas of the hydrogen peroxide gas and the carrier hot air can rise snakingly or swirlingly can be applied. Further the shield plate is not limitted to the disk-like shield. The drop splash removing apparatus is not limitted to the construction in which the plural shield plates provided with the plural holes are arranged to form stairs.

The upper portion of the upper inner tube 10 opens to the upper inner surface of the inner cylinder 7 and a lower inner tube 11 is connected with the lower portion of the upper inner tube 10. The lower inner tube 11 passes through an inner path 12C of the evaporation unit 12C and an exhaust port 13 for the carrier hot air is provided in the lower portion of the lower inner tube 11.

On the other hand, the evaporation unit 12 is provided inside the lower outer cylinder 3, which evaporation unit 12 comprising an upper cylinder portion 12A and an lower cylinder portion 12B (Fig 3) having a diameter less than that of the upper cylinder portion and the inner path 12C is vertically provided in the inner side of the upper cylinder portion 12A and the lower cylinder portion 12B. Plural concaved cylindrical vaporization part 14 are provided on the upper surface of the cylindrical portion 12A. As described in Fig. 4, a cylindrical net 15 is provided about the surrounding of the concaved cylindrical vaporization part 14. The vaporization part 14 is not limitted to the concaved cylindrical unit, any dished unit can be applied. Plural heaters 16 are arranged to form stages in the lower cylinder portion 12B and lead wires 17 are connected with the heater 16. A space 22 is provided below the concaved cylindrical vaporization part 14 inside the evaporation unit 12 and a thermo sensor 18 is inserted to the upper portion thereof from the outside of the lower outer cylinder 3.

A vaporization condition can be measured by the thermo sensor 18 substantially equal to the condition of the concaved cylindrical vaporization part 14. In the part of the space 22 belonging to the upper cylinder portion 12A, it can be filled with a filler having good coefficient of thermal conductivity and also it can be integrally molded with the same material as the evaporation unit 12. In this case, as described in Fig. 3, the space of the upper cylinder portion 12A is used only as a hole for the thermo sensor. In the part of the space 22 belonging to the lower cylinder portion 12B, heat insulating material 21 is filled.

The hydrogen peroxide liquid is supplied from a quantitative apparatus ( not shown ) through plural supply tubes 19. As described in Fig. 1, dropping nozzles 20 are connected with the end of the supply tube 19 and each dropping nozzle passes through the lower outer cylinder 3 and is turned down above the concaved cylindrical vaporization part 14.

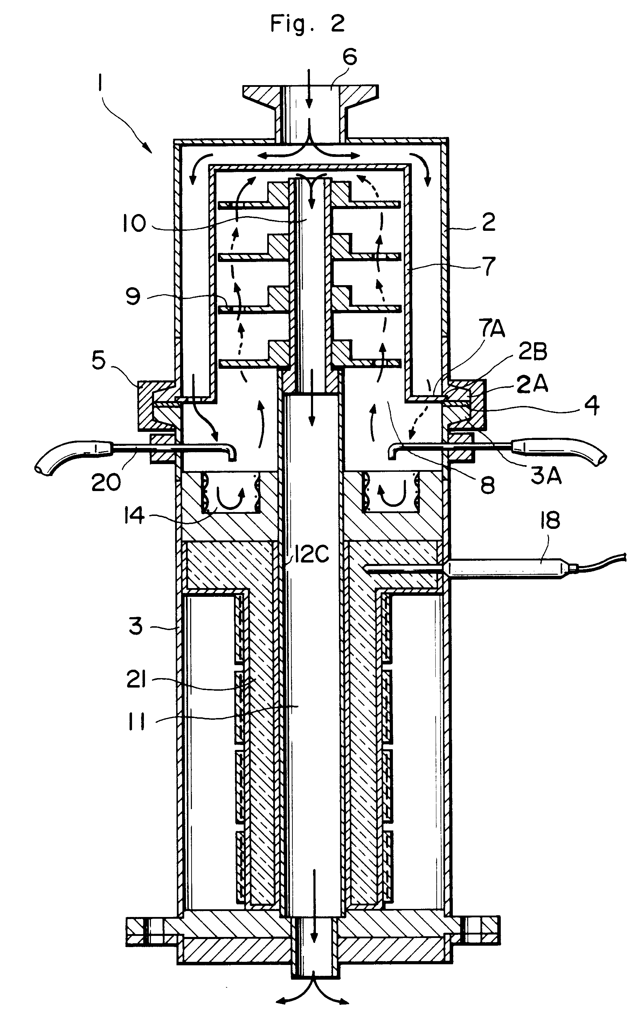

The operation of the disinfectant vaporizing apparatus according to the present invention is described based on Fig. 1 and 2. The pre-heated carrier gas at high temperature is applied to the disinfectant vaporizing apparatus from an inlet port 6. The heated carrier gas lowers along the interval between an upper outer cylinder 2 and an inner cylinder 7 to reach an evaporation chamber 8 through an opening 7B.

On the other hand, disinfectant applied from a quantitative supply apparatus is dropped on a dished vaporization part from a dropping nozzle 20 through a supply tube 19. An evaporation unit 12 is heated by a heater means 16. Because the dished vaporization part is heated by heat transmitted through the outer or inner surface of the evaporation unit 12, the drop of the disinfectant dropped on the dished vaporization part is heated to evaporate.

When heating temperature of the disinfectant drop becomes over a predetermined temperature, a membrane appears between the drop and the evaporation surface to subject to float droplet particles and while the coefficient of heat transfer decreases, the evaporation time increases, namely spheroidal phenomenon occurs. Therefore, a cylindrical net 15 may be provided about the surrounding of the dished, for example concaved cylindrical evaporation part 14. With this, the drop is broken into parts by the cylindrical net 15, resulting in shorter evaporation time.

Disinfection gas heated and vaporized on the dished evaporation part is carried to an opening of an upper inner tube 10 through a droplet splash removing apparatus, accompanied with the heated carrier gas.

The droplet splash removing apparatus is provided with plural shield disks arranged to form stairs, said disks provided with plural holes, said holes adjacent to each other of said plural holes 9A of said shield disk 9 should be arranged staggeringly, additionally the plural holes provided on the plural shield disks 9 adjacent to each other should be arranged not to overlap each other to the vertical direction. Then the mix gas of the disinfection gas and the heated carrier gas rise snakingly or swirlingly. And the dropwise or splashwise disinfectant cannot rise therewith and combine each other into large drop, resulting in falling. Further since the mix gas of the disinfection gas and the heated carrier gas is snaked or swirled, the resulting mix gas has a uniform density.

The mix gas of the disinfection gas and the heated carrier gas carried to the opening of the upper inner tube 10 from the drop splash removing apparatus descends through the upper inner tube 10 and the lower inner tube 11 to be supplied to the disinfection apparatus directly from an exhaust port 13 or through an induction pipe ( not shown ).

While the invention has been particularly shown and described with reference to preferred embodiment thereof, it will be understood by those skilled in the art that the foregoing and other changes in form and details can be made therein without departing from the spirit and scope of the invention.

## Claims

1. A gas generator for supplying disinfectant gas, comprising
(a) a vaporization chamber (8) for enriching a heated carrier gas with a gaseous disinfectant,
(b) a vaporizer (12) which is supplied by at least one dropping nozzle (20) with a vaporizable liquid,
characterized in that
(c) the vaporizer (12) in its upper part has a disk-like portion (12A), which is heatable,
(d) in the upper surface of the disk-like portion (12A) a plurality of cavities (14) are disposed, in which liquid disinfectant is vaporized,
(e) above each cavity (14) at least one dropping nozzle (20) is provided and
(f) a labyrinthal arrangement (9) positioned between the disk-like portion (12A) and the opening of an inner tube (10) are provided.

2. A gas generator according to claim 1,
characterized in that
(a) above the disk-like portion (12A) a cylindrical outer shell (2) carrying an inlet port (6) for the carrier gas at its upper end, and an inner cylindrical shell (7) are arranged, the latter having a closed upper end, an opened lower end (7A) and is contained within said outer shell such that a space (7C) is provided between the outer and the inner shell (2; 7),
(b) said space (7C) is connected to said inlet port (6) and to openings (7A) adjacent to said cavities (14),
(c) an elongated inner tube (10) is disposed within said inner shell (7) with the upper end of said tube covered by the inner shell (7),
and
(d) the labyrinthal arrangement (9) consists of perforated plates (9) which are disposed around the inner tube (10) above the cavities (14) and inside the shell (7) to remove droplets from the stream of carrier gas inside the vaporization chamber (8).

3. A gas generator according to claim 2, characterized in that the perforated plates (9) are disposed in a parallel staggered arrangement.

4. A gas generator according to claims 2 and 3, characterized in that the perforations (9a) in the plates (9) are arranged in non-overlapping vertical positions, see from one plate (9) to another.

5. A gas generator according to one of the claims 1-4, characterized in that the cavities (14) have a cylindrical shape and are carrying a cylindrical net (15) covering the inner mantle surface of the cavities.

6. A gas generator according to any of the preceding claims, characterized in that the inner tube (10) opens to the upper inner surface of the inner cylinder (7) and is further connected to the exhaust port (13) through the lower inner tube (11).

7. A gas generator according to claims 1-6,
characterized in that the heating means (16) for the vaporizer (12) are disposed beneath the disk-like portion (12A), and that a lower end of the inner tube (11) extends through said heating means (16).

8. A gas generator according to claim 7, characterized in that said heating means (16) have an elongated lower portion (12B) and in that said lower inner tube (11) extends through said elongated lower portion (12B).

9. A gas generator according to one of the preceding claims, wherein a thermosensor (18) is disposed in the upper cylinder portion (12A) of the vaporizer (12).

## Patentansprüche

1. Gasgenerator zur Erzeugung eines desinfizierenden Gases,
umfassend
(a) eine Verdampfungskammer (8) zur Anreicherung eines erwärmten Trägergases mit einem gasförmigen Desinfektionsmittel,
(b) einen Verdampfer (12), der durch wenigstens eine Tropfdüse (20) mit einer verdampfbaren Flüssigkeit versorgt wird,
dadurch gekennzeichnet, daß
(c) der Verdampfer (12) in seinem oberen Bereich einen scheibenartigen Abschnitt (12A) hat, der aufheizbar ist,
(d) auf der Oberseite des scheibenartigen Abschnittes (12A) eine Vielzahl von Mulden (14) angeordnet ist, in denen das flüssige Desinfektionsmittel verdampft wird,
(e) oberhalb jeder Mulde (14) wenigstens eine Tropfdüse (20) vorgesehen ist, und
(f) eine Labyrinth-Vorrichtung (9) zwischen dem scheibenartigen Abschnitt (12A) und der Öffnung eines Innenrohrs (10) vorgesehen ist.

2. Gasgenerator nach Anspruch 1, dadurch gekennzeichnet, daß
(a) oberhalb des scheibenartigen Abschnittes (12A) ein zylindrisches äußeres Gehäuse (2), das einen Einlaß (6) für das Trägergas an seinem oberen Ende aufweist, und ein Innengehäuse (7) vorgesehen sind, wobei letzteres ein geschlossenes oberes Ende und ein offenes unteres Ende (7A) aufweist und vom äußeren Gehäuses umschlossen ist, wobei ein Zwischenraum (7C) zwischen dem äußeren und dem inneren Gehäuse (2; 7) verbleibt,
(b) der Zwischenraum (7C) mit dem Einlaß (6) und mit Öffnungen (7A) verbunden ist, die sich im Bereich der Mulden (14) befinden,
(c) ein längliches Innenrohr (10) im Innengehäuse (7) angeordnet ist, wobei das obere Ende des Innenrohres von dem Innengehäuse (7) abgedeckt ist, und
d) daß die Labyrinth-Anordnung (9) aus perforierten Platten (9) besteht, die um das Innenrohr (10) angeordnet sind, und zwar oberhalb der Mulden (14) und innerhalb des Gehäuses (7), um Tröpfchen aus dem Trägergasstrom innerhalb der Verdampfungskammer (8) zu entfernen.

3. Gasgenerator nach Anspruch 2, dadurch gekennzeichnet, daß die perforierten Platten (9) parallel liegend übereinander angeordnet sind.

4. Gasgenerator nach Anspruch 2 und 3, dadurch gekennzeichnet, daß die Perforationen (9A) in den Platten (9) so angeordnet sind, daß sie - gesehen von einer Platte (9) zur anderen - in nicht überlappender vertikaler Position liegen.

5. Gasgenerator nach einem der Ansprüche 1 bis 4, dadurch gekennzeichnet, daß die Mulden (14) zylindrisch sind und ein zylindrisches Netz (15) tragen, das die innere Mantelfläche der Mulden abdeckt.

6. Gasgenerator nach einem der vorhergehenden Ansprüche, dadurch gekennzeichnet, daß das Innenrohr (10) sich zur oberen Innenfläche des inneren Zylinders (7) öffnet und darüberhinaus verbunden ist mit dem Auslaß (13) durch das untere Innenrohr (11).

7. Gasgenerator nach Ansprüchen 1 bis 6, dadurch gekennzeichnet, daß die Heizung (16) für den Verdampfer (12) unterhalb des scheibenartigen Abschnittes (12A) angeordnet ist, und daß ein unteres Ende des Innenrohrs (11) sich durch die Heizung (16) hindurchstreckt.

8. Gasgenerator nach Anspruch 7, dadurch gekennzeichnet, daß die Heizung (16) einen länglichen unteren Abschnitt (12B) besitzt und daß das genannte Innenrohr (11) sich durch den länglichen unteren Abschnitt (12B) erstreckt.

9. Gasgenerator nach einem der vorherigen Ansprüche, gekennzeichnet durch einen Thermofühler (18), der in dem oberen Zylinderabschnitt (12A) der Verdampfers (12) angeordnet ist.

## Revendications

1. Générateur de gaz pour délivrer un désinfectant gazeux, comprenant:
(a) une chambre de vaporisation (8) pour l'enrichissement d'un gaz porteur chauffé, par un désinfectant gazeux,
(b) une unité de vaporisation (12) qui est alimentée par au moins un injecteur de gouttelettes (20) avec un liquide pouvant être vaporisé,
caractérisé en ce que
(c) l'unité de vaporisation (12) est pourvue, dans sa partie supérieure, d'une partie en forme de disque (12A), qui peut être chauffée,
(d) sur la face supérieure de la partie en forme de disque (12A), une pluralité de cavités (14), dans lesquelles le liquide désinfectant est vaporisé, est disposée,
(e) au-dessus de chaque cavité (14) au moins un injecteur de gouttelettes 20 est disposé, et
(f) un dispositif en labyrinthe (9) disposé entre la partie en forme de disque (12A) et l'ouverture d'un tube interne (10), est prévu.

2. Générateur de gaz selon la revendication 1,
caractérisé en ce que
(a) au-dessus de la partie en forme de disque (12A) une enveloppe externe cylindrique (2), disposant d'une embouchure d'entrée (6) destinée au gaz porteur à son extrémité supérieure, et une enveloppe interne cylindrique (7) sont disposées, cette dernière dispose d'une extrémité supérieure fermée, d'une extrémité inférieure ouverte (7A) et est contenue dans ladite enveloppe externe de façon à ce qu'un espace (7C) soit créé entre les enveloppes externe et interne (2; 7),
(b) ledit espace (7C) est relié à ladite embouchure d'entrée (6) et aux orifices (7A) adjacents auxdites cavités (14),
(c) un tube interne allongé (10) est disposé à l'intérieur de ladite enveloppe interne (7) de façon à ce que l'extrémité supérieure dudit tube soit recouverte par l'enveloppe interne (7),
et
(d) le dispositif en labyrinthe (9) consiste en plaques perforées (9) qui sont disposées autour du tube interne (10) au-dessus des cavités (14) et à l'intérieur de l'enveloppe (7) afin de supprimer les gouttelettes de l'écoulement de gaz porteur de la chambre de vaporisation (8).

3. Générateur de gaz selon la revendication 2, caractérisé en ce que les plaques perforées (9) sont disposées en étages parallèles.

4. Générateur de gaz selon l'une des revendications 2 et 3, caractérisé en ce que les perforations (9a) dans les plaques (9) sont disposées pour éviter un recouvrement vertical, en regardant d'une plaque (9) vers une autre.

5. Générateur de gaz selon l'une des revendications 1 à 4, caractérisé en ce que les cavités (14) sont de forme cylindrique et supportent une grille cylindrique (15) recouvrant la surface de la paroi interne des cavités.

6. Générateur de gaz selon l'une quelconque des revendications précédentes, caractérisé en ce que le tube interne (10) débouche sur la surface interne supérieure du cylindre interne (7) et en ce qu'il est en outre raccordé à l'embouchure d'évacuation (13) à travers le tube interne inférieur (11).

7. Générateur de gaz selon l'une des revendications 1 à 6, caractérisé en ce que les moyens de chauffage (16) de l'unité de vaporisation (12) sont disposés au-dessous de la partie en forme de disque (12A), et en ce qu'une extrémité inférieure du tube interne (11) s'étend à travers lesdits moyens de chauffage (16).

8. Générateur de gaz selon la revendication 7, caractérisé en ce que lesdits moyens de chauffage (16) présentent une partie inférieure allongée (12B) et en ce que ledit tube interne inférieur (11) s'étend à travers ladite partie inférieure allongée (12B).

9. Générateur de gaz selon l'une des revendications précédentes, dans lequel un capteur thermo-sensible (18) est disposé dans la partie cylindrique supérieure (12A) de l'unité de vaporisation (12).
